# EUROPEAN PATENT APPLICATION

(11) **EP 0 885 612 A1**
(43) Date of publication of application: **23.12.1998**
(21) Application number: 98830353.3
(22) Date of filing: 05.06.1998
(51) Int. Cl.: A61K 31/535

(54) **Pirenoxine for the topical treatment of inflammatory conditions**

(30) Priority: 06.06.1997 IT MI971341
(71) Applicant: Pharmaconsult s.a.s., 95124 Catania (IT)
(72) Inventor: Drago, Filippo, 95124 Catania (IT)
(74) Representative: Banchetti, Marina

(57) **Abstract**

Pirenoxine, i.e. 1-hydroxy-5-oxo-5H-pyrido-[3,2-a]-phenoxazine-3-carboxylic acid (also referred to as pirfenoxone) or a pharmaceutically acceptable salt thereof, a known anti-cataract agent, is used as an active ingredient for the topical treatment of inflammatory conditions. More specifically, pirenoxine is used for the topical treatment of ophthalmic inflammatory conditions such as corneal and conjunctival inflammations, uveitis, ocular oedema.

## Description

The present invention relates to the use pirenoxine or of a pharmaceutically acceptable salt thereof for the topical treatment of inflammatory conditions. More specifically, this invention concems the use of pirenoxine and its salts as anti-inflammatory agents for topical ophthalmic application.

Pirenoxine, i.e. 1-hydroxy-5-oxo-5H-pyrido-[3,2-a]-phenoxazine-3-carboxylic acid (also referred to as pirfenoxone), is a known compound having the following formula: 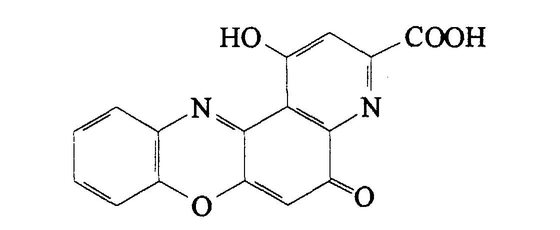 which is used in ophthalmology, usually in the form of the corresponding sodium salt, for the treatment of cataract. The latter is an abnormal progressive condition of the lens of the eye, characterised by increasing loss of transparency. As it is known, cataracts are most often caused by degenerative changes, often occurring after 50 years of age, while less often they may be caused by trauma or by exposure to poisons. At the onset, vision is blurred, then bright lights glare diffusely and distortion and double vision may develop. Eventually, if the cataract is untreated, sight is totally lost. Besides the surgical treatment, that becomes finally necessary and involves the excision of the lens (with or without the surgical insertion of an intraocular lens), cataracts may be treated by the administration of pirenoxine to the eye, normally in the form of an ophthalmic solution, i.e. eye drops.

The activity of pirenoxine in inhibiting the formation of lens opacities has been attributed to at least three different mechanisms of action: (a) inhibition of the oxidising activity that quinone molecules exert on the proteins of the lens, by binding to their -SH groups; (b) activation and normalisation of the cationic pump function of the crystalline capsule; (c) inhibition of the sorbitol synthesis and resulting reduction of the osmotic damage deriving from the accumulation of this substance (Iwata S., J. Pharmac. Soc. Jap. 1964; 84: 435-440; Ikemoto F., Fukui S., Iwata S., In: Proc. 50th Congr. Pharmacol. Soc. Jap., Kanto Region, 1974; Korte I., Hockwin O., Ohrloff C., Ophthalmic Res. 1979; 11: 123-125).

More recently, some further pharmacological effects of pirenoxine have been evidenced, i.e., (d) dose-dependent increase of the soluble proteins and of the sulfur-containing amino acids, and (e) dose-dependent increase of glutathione at the crystalline lens level (Drago F., D'Agata V., Marino V., Marino A., Blasco G., Pharmacol. Res. 1995; 31: 325-329).

As a follow-up of the above studies on the biological activity of pirenoxine it has been found, in accordance with the present invention, that pirenoxine, besides being active in the treatment of cataracts, is also markedly active in counteracting or reducing inflammation, specifically as a topical anti-inflammatory agent suitable for ophthalmic use. Actually, it has been shown experimentally that pirenoxine, when applied topically, in particular to the eye, is active against inflammation at a level comparable with that of other known anti-inflammatory agents, specifically non-steroidal anti-inflammatory agents.

The main advantage that pirenoxine appears to possess with respect to many known non-steroidal anti-inflammatory agents is that it does not appear to show, when administered to the eye, the same typical side effects of this group of active ingredients, i.e. pain and irritation of the conjunctiva. As a matter of fact, pirenoxine shows no analogy with the group of non-steroidal anti-inflammatory agents as far as its chemical structure is concerned, nor it shows any analogy as concems its possible mechanism of action. The anti-inflammatory activity of pirenoxine is very likely to be ascribed to an inhibitory action on the oxidative catabolism of arachidonic acid, which leads to the production of prostaglandins. An additional clinical advantage of pirenoxine lies in the manifold action as an antioxidant and against protein denaturation that this drug exerts on the lens.

Therefore, the present invention specifically provides the use of 1-hydroxy-5-oxo-5H-pyrido-[3,2-a]-phenoxazine-3-carboxylic acid (pirenoxine) or of a pharmaceutically acceptable salt thereof for the manufacture of a medicament for the topical treatment of inflammatory conditions and, in particular, for the treatment of inflammations of the ocular region. Specifically, the indications for the proposed use include the treatment of inflammatory conditions such as corneal and conjunctival inflammations, uveitis and ocular oedema.

The ophthalmic preparations of the present invention preferably contain the active ingredient, i.e. pirenoxine or a pharmaceutically applicable salt thereof, in an amount from 0.0001% to 0.01% by weight, expressed as the free acid form. More suitably, the said medicaments contain from 0.001% to 0.005% by weight of pirenoxine, expressed as the free acid form, an optimal concentration being 0.001% by weight. Most suitably, said pirenoxine is in the form of the corresponding sodium salt. When used as eye drops with 0.001% by weight of active ingredient, the preparation according to the invention may be administered, to obtain the desired anti-inflammatory effect, at a dosage of one-two drops from two to three times a day, preferably two drops three times a day. In general, the dosage and frequency of administration of the product will depend of the seriousness of the condition to be treated and on the general conditions of the patient.

The topical ophthalmic medicament containing pirenoxine or a salt thereof may be, in general, in the form of an aqueous solution or suspension for eye drops or in the form of an emulsion, an ointment, a gel or a cream. Preferably, the product is administered in the form of an aqueous ophthalmic solution. In view of the instability of the active ingredient, pirenoxine is normally formulated, in the medicinal products already in use for the treatment of cataracts, as a two-component preparation, wherein a first component comprises lyophilised pirenoxine, in the sodium salt form, together with an ocularly acceptable carrier, and the second component comprises an ocularly acceptable aqueous vehicle or carrier. The two components are reconstituted before use and the solution thus obtained may generally be kept at room temperature for about two weeks without degradation.

In general, the compositions containing pirenoxine or a salt thereof according to the invention may be formulated in accordance with the known art, for example according to the teachings given in "Remington's Pharmaceutical Sciences Handbook", Hack Publ. Co., U.S.A.. Usually, one or more tonicity adjusting agents should be added , so as to give the solution a correct value of osmolarity. Any one of the products currently employed in the art as tonicity agents may be used, such as, for instance, sodium chloride, potassium chloride, mannitol, dextrose, boric acid, propylene glycol. The preparation may also comprise acids or bases as pH adjusting agents and/or buffers, such as, e.g., the monosodium phosphate - disodium phosphate system, the sodium borate - boric acid system or the sodium succinate - succinic acid system. For good tolerability by the eye, the pH should be comprised between 4.5 and 8.5.

Further, the composition should also comprise preservatives and antimicrobial agents, such as benzalkonium chloride, sodium merthiolate or thimerosal, methyl-, ethyl- and propyl paraben, chlorobutanol, as well as chelating or sequestering agents such as the edetates or EDTA. In the event that the product is packaged in unit-dose containers the presence of preservatives may be avoided, but when the product is in multiple dose containers, e.g. eye drop bottles containing from 5 to 15 ml, the use of preservatives is necessary.

In addition, the ophthalmic preparation may include further optional ingredients, such as thickening agents, antioxidants, stabilisers, surface-active agents, etc.. By way of example only, the composition of a commercial product already in use for the treatment of cataracts is described below. The formulation may be suitable also for use as ocular anti-inflammatory medicament, possibly with a reduction of the concentration of active ingredient.

### EXAMPLE

### Lyophilised sodium pirenoxine formulation

The dry powder component of the product has the following composition, wherein the amounts are given for the reconstitution in a 7 ml solution:

| | |
|---|---|
| sodium pirenoxine | mg 0.376 |
| (corresponding to mg 0,350 of pirenoxine) | |
| taurine | mg 34.34 |

Taurine, which is employed in the formulation as an antioxidant, is dissolved in deionised water, while sodium pirenoxine is dissolved in a separate amount of deionised water. The two solutions are sterilised by filtration and then mixed together, and subjected to the lyophilisation procedure.

The aqueous solvent component has the following composition:

| | |
|---|---|
| polyvinyl alcohol | mg 98 |
| succinic acid | mg 2.31 |
| sodium succinate · 6H₂O | mg 89.215 |
| sodium chloride | mg 34.3 |
| benzalkonium chloride | mg 0.175 |
| sodium edetate | mg 0.89 |
| deionised water | q.s. to 7 ml |

Besides the ingredients that have already been mentioned in the foregoing description, the above formulation contains PVA as thickening agent. The pH of the solvent component is 6. The formulation is prepared by first stirring and dissolving in water all of the ingredients but benzalkonium chloride. After complete dissolution of all products benzalkonium chloride is added while keeping stirring, and the mixture is sterilised by filtration.

The reconstituted product has a pH of 6-6.3.

### Anti-inflammatory activity tests

In order to evaluate the performance of pirenoxine as an anti-inflammatory agent, some experiments were carried out on animal models as summarised below.

### 1. Rat paw phlogosis model

Male Wistar rats weighing about 200 g were used. After a period of suitable stabling, oedema was induced in the animals by an injection of a solution 0.5% by weight of carrageenin in the plantar zone of the right paw. The animals had been divided into three groups, which were treated as follows:
the animals of the first group were given, by sub-cutaneous injection one hour before the phlogosis induction, sodium pirenoxine in an amount corresponding to 0.5 mg/kg of the free acid, dissolved in a suitable solvent; the animals of the second group received an injection of sodium diclofenac in an amount corresponding to 0.5 mg/kg of the free acid, dissolved in a suitable solvent;
the animals of the third group received an injection of plain physiologic solution.

The development of the inflammation was evaluated by means of a plethysmograph after 20, 30, 60, 120 e 240 minutes from the induction of the phlogosis. The test results are shown in the following table.

**TABLE 1**

| Acute anti-inflammatory effect on the rat paw oedema | | | | | |
|---|---|---|---|---|---|
| | Time from the carrageenin injection (hours) | | | | |
| Experimental groups (No. of animals) | 20 | 30 | 60 | 120 | 240 |
| physiological solution (5) | 3.40 | 3.20 | 2.60 | 2.00 | 1.20 |
| pirenoxine (5) | 3.40 | 3.00 | 2.40 | 2.10 | 1.10 |
| diclofenac (5) | 3.00 | 2.60* | 1.80* | 1.60* | 0.80* |
| The above values are average values (S.E. omitted as it is lower than 0.1) of the paw volume expressed in mmHg. | | | | | |

| | | | | | |
|---|---|---|---|---|---|
| *: The difference vs. the group treated with physiological solution is statistically significant (p < 0.05 Dunnett's test by multiple comparisons) | | | | | |

As is shown by the above data, pirenoxine has no significant protective action against the carrageenin-induced inflammation in the rat paw model.

### 2. First rabbit ocular phlogosis model

Male New Zealand albino rabbits weighing about 2 kg were used. After a period of suitable stabling, an aseptic phlogosis was induced in the right eye of the animals by a sub-conjunctival injection of a solution 0.5% by weight of formaldehyde. The animals had been divided into three groups, which were treated as follows:
the animals of the first group were given sodium pirenoxine eye drops, at a concentration corresponding to 0.001% of the free acid, two drops instilled in the conjunctival sac every 8 hours;
the animals of the second group received a similar treatment with sodium diclofenac eye drops, at a concentration corresponding to 0.1% of the free acid, two drops instilled in the conjunctival sac every 8 hours;
the animals of the third group received a similar treatment with physiologic solution, two drops instilled in the conjunctival sac every 8 hours.

The treatment took place 10 minutes after the sub-conjunctival injection of the inflammatory agent and was carried out for 15 days.

The development of the inflammation was studied by evaluating the following parameters:
- conjunctival hyperaemia;
- conjunctival chemosis;
- corneal oedema;
- Tyndall effect;
- cellularity in the anterior chamber.

The above parameters were evaluated according to the following scores: (0), absent; (1), light; (2), moderate; (3), serious.

Immediately after the sacrifice, some samples of aqueous were taken by means of heparinised syringes. A staining in Wright-Giemsa was carried out and then a differential leukocytes count under microscope (magnification 400x). The number of red and white series cells in the aqueous was determined with the haemocytometric method. The test results are shown in the following table.

**TABLE 2**

| Anti-inflammatory effect on the formaldehyde-induced ocular phlogosis in rabbit | | | | |
|---|---|---|---|---|
| Experimental groups (No. of animals) | hyperaemia | chemosis | corneal oedema | cellularity |
| physiological solution (5) | 1.00 | 0.20 | 1.00 | 1.00 |
| pirenoxine (5) | 0.40* | 0.20 | 0.40* | 0.40* |
| diclofenac (5) | 0.40* | 0.00* | 0.00* | 0.00* |
| The above values are average values (S.E. omitted as it is lower than 0.1). | | | | |

| | | | | |
|---|---|---|---|---|
| *: The difference vs. the group treated with physiological solution is statistically significant (p < 0.05 Dunnett's test by multiple comparisons) | | | | |

In the frame of the above test, a quantitative evaluation of the total proteins of the aqueous and of the vitreous humour was also carried out, by means of a phenolic reagent. The myeloperoxidase activity (i.e., a marker for the contents of neutrophiles in the uveal tissue) was determined by measuring the change of the absorbance at 460 nm of a mixture of tissue homogenate, buffer, o-diansidine dihydrochloride and hydrogen peroxide. The prostaglandins and leukotrienes contents were determined in the aqueous by radio-immunologic method.

The data obtained in the above experimentation actually show an anti-inflammatory activity of pirenoxine comparable with the activity of diclofenac. The parameters that appeared to be more responsive to the action of pirenoxine are hyperaemia, chemosis and the cellularity in the anterior chamber.

### 3. Second rabbit ocular phlogosis model

The above anti-inflammatory activity of pirenoxine was also confirmed in a model of ocular phlogosis in rabbit induced by Nd:YAG laser. The laser treatment was carried out under general anaesthesia. The energy was administered through a silica fibre of 600 µm diameter. Apart from the different method of inducing inflammation, the scheme of the experimentation was the same as in the previous test. The results are shown in the following table.

**TABLE 3**

| Anti-inflammatory effect on the Nd:YAG laser-induced ocular phlogosis in rabbit | | | | |
|---|---|---|---|---|
| Experimental groups (No. of animals) | hyperaemia | chemosis | corneal oedema | cellularity |
| physiological solution (5) | 1.00 | 0.20 | 1.00 | 0.40 |
| pirenoxine (5) | 0.40* | 0.20 | 0.00* | 0.40 |
| diclofenac (5) | 0.40* | 0.20 | 0.00* | 0.40 |
| The above values are average values (S.E. omitted as it is lower than 0.1). | | | | |

| | | | | |
|---|---|---|---|---|
| *: The difference vs. the group treated with physiological solution is statistically significant (p < 0.05 Dunnett's test by multiple comparisons) | | | | |

The present invention has been disclosed with particular reference to some specific embodiments thereof, but it should be understood that modifications and changes may be made by the persons skilled in the art without departing from the scope of the invention as defined in the appended claims.

## Claims

1. Use of 1-hydroxy-5-oxo-5H-pyrido-[3,2-a]-phenoxazine-3-carboxylic acid (pirenoxine) or of a pharmaceutically acceptable salt thereof for the manufacture of a medicament for the topical treatment of inflammatory conditions.

2. Use according to claim 1, wherein said medicament is a topical ophthalmic medicament.

3. Use according to claim 2, wherein the said medicament is indicated for the treatment of inflammatory conditions chosen from the group consisting of comeal and conjunctival inflammations, uveitis, ocular oedema.

4. Use according to claims 2 or 3, wherein said medicament contains from 0.0001% to 0.01% by weight of pirenoxine, expressed as the free acid form.

5. Use according to claim 4, wherein said medicament contains from 0.001% to 0.005% by weight of pirenoxine, expressed as the free acid form.

6. Use according to any one of claims 2-5, wherein said pirenoxine is in the form of the corresponding sodium salt.

7. Use according to any one of claims 2-6, wherein said topical ophthalmic medicament is in the form of an aqueous solution or suspension for eye drops, or in the form of an emulsion, an ointment, a gel or a cream.

8. Use according to claim 7, wherein said aqueous solution is obtained by reconstitution of a two-component preparation, wherein a first component comprises lyophilised pirenoxine, in the sodium salt form, together with an ocularly acceptable carrier, and the second component comprises an ocularly acceptable aqueous vehicle or carrier.
